(19) Europäisches Patentamt
European Patent Office
Office européen des brevets

(11) Veröffentlichungsnummer : **0 089 528**
**B1**

(12) # EUROPÄISCHE PATENTSCHRIFT

(45) Veröffentlichungstag der Patentschrift : **27.05.87**

(51) Int. Cl.⁴ : **C 07 D237/04**, C 07 D237/26, A 61 K 31/50

(21) Anmeldenummer : **83102127.4**

(22) Anmeldetag : **04.03.83**

(54) **6-Aryl-4,5-dihydro-3(2H)-pyridazinone,ihre Herstellung und Verwendung.**

(30) Priorität : **13.03.82 DE 3209159**

(43) Veröffentlichungstag der Anmeldung : **28.09.83 Patentblatt 83/39**

(45) Bekanntmachung des Hinweises auf die Patenterteilung : **27.05.87 Patentblatt 87/22**

(84) Benannte Vertragsstaaten : **AT BE CH DE FR GB IT LI NL SE**

(56) Entgegenhaltungen :
**EP-A- 0 000 113**
**EP-A- 0 012 366**
**EP-A- 0 107 735**
**DE-A- 2 304 977**

(73) Patentinhaber : **BASF Aktiengesellschaft**
**Carl-Bosch-Strasse 38**
**D-6700 Ludwigshafen (DE)**

(72) Erfinder : **Rossy, Phillip A., Dr.**
**Van-Leyden-Strasse 17**
**D-6700 Ludwigshafen (DE)**
Erfinder : **Thyes, Marco, Dr.**
**Thorwaldsenstrasse 1**
**D-6700 Ludwigshafen (DE)**
Erfinder : **Franke, Albrecht, Dr.**
**Mandelring 11**
**D-6706 Wachenheim (DE)**
Erfinder : **Koenig, Horst, Dr.**
**Pariser Strasse 4**
**D-6700 Ludwigshafen (DE)**
Erfinder : **Gries, Josef, Dr.**
**Roemerweg 43**
**D-6706 Wachenheim (DE)**
Erfinder : **Lehmann, Hans Dieter, Dr.**
**Im Hefen 15**
**D-6945 Hirschberg (DE)**
Erfinder : **Lenke, Dieter, Dr.**
**Kekuleplatz 1**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 089 528 B1

**Beschreibung**

Die Erfindung betrifft neue 6-Aryl-4,5-dihydro-3(2H)-pyridazinone, Verfahren zu deren Herstellung und deren Verwendung bei der Bekämpfung von Krankheiten.

Es sind bereits eine Reihe von Phenyl-4,5-dihydro-3(2H)-pyridazinonen bekannt (DE-OS 1 670 158, DE-OS 2 123 246, DE-OS 2 150 436, DE-OS 2 157 453, DE-OS 2 304 977, DE-OS 2 727 481, DE-OS 2 854 191, DE-OS 2 854 475, DE-OS 3 022 176, DE-OS 3 022 177, DE-OS 3 033 702, JP-OS 53.124-279, US-PS 3 824 271, US-PS 3 888 901). Diese Verbindungen sollen blutdrucksenkende, entzündungshemmende, cardiovaskuläre, antiphlogistische, coronarerweiternde, antiallergische, membranstabilisierende und/oder thrombozytenaggregationshemmende Eigenschaften besitzen.

Zum Stand der Technik gemäss Art. 54 (3) EPÜ gehören die blutdrucksenkenden und thrombozytenaggregationshemmenden 6-(4-Acylaminophenyl)-4,5-dihydro-3(2H)-pyridazinonderivate gemäss EP-A-107 735.

Es wurde nun gefunden, daß 6-Aryl-4,5-dihydro-3(2H)-pyridazinone der Formel

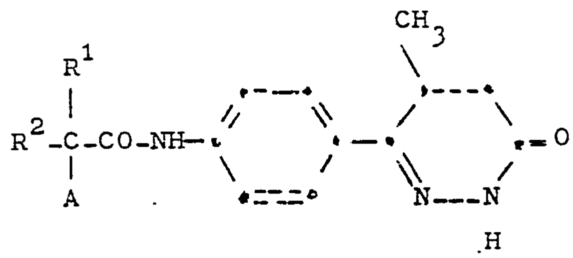

(I)

in der

$R^1$ und $R^2$ gleich oder verschieden sind und Wasserstoffatome oder $C_{1-6}$-Alkylgruppen darstellen und

A eine $C_{1-6}$-Alkoxy-, $C_{1-6}$-Alkylmercapto-, $C_{2-7}$-Alkylaminocarbonyloxy-, $C_{1-6}$-Alkylsulfonyloxy-, Carboxyl-, $C_{2-7}$-Alkoxycarbonyl- oder Cyanogruppe darstellt,

wertvolle pharmakologische Eigenschaften besitzen.

Die Angabe der Zahl der C-Atome für die Substituenten A bezieht sich auf die Gesamtzahl der C-Atome in dem betreffenden Rest.

Als besonders günstig haben sich die Verbindungen erwiesen, in denen $R^1$ ein Wasserstoffatom oder eine Methylgruppe, $R^2$ ein Wasserstoffatom oder eine Methylgruppe und A eine Methoxy- oder Cyanogruppe darstellen.

Die neuen Verbindungen lassen sich herstellen, indem man

a) eine Verbindung der Formel II

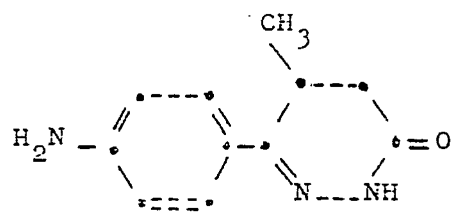

(II)

mit einem Carbonsäurehalogenid der Formel III

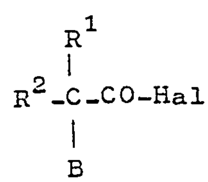

(III)

worin $R^1$ und $R^2$ die angegebene Bedeutung besitzen und B eine $C_{2-7}$-Alkanoyloxy-, $C_{1-6}$-Alkoxy-, $C_{1-6}$-Alkylmercapto-, Carboxyl-, $C_{2-7}$-Alkoxycarbonylgruppe oder Cyanogruppe darstellt, umsetzt und in den so erhaltenen Verbindungen eine gegebenenfalls enthaltene Acyloxygruppe verseift und die so erhaltenen Hydroxyverbindungen mit einem reaktionsfähigen Derivat einer $C_{1-6}$-Alkylsulfonsäure oder einem $C_{2-7}$-Alkylisocyanat umsetzt oder gegebenenfalls eine Carboxylgruppe verestert, oder

b) — falls A eine $C_{1-6}$-Alkoxy-, $C_{1-6}$-Alkylmercapto- oder Cyanogruppe ist — in einer Verbindung der Formel (IV)

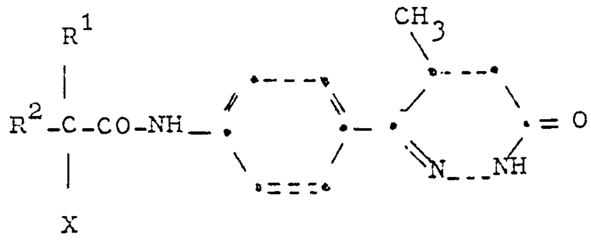

(IV)

in der $R^1$ und $R^2$ die oben angegebenen Bedeutungen haben und X für ein Chlor-, Brom- oder Iodatom oder eine Alkylsulfonyloxygruppe steht, den Rest X gegen eine $C_{1-6}$-Alkoxy-, $C_{1-6}$-Alkylmercapto- oder Cyanogruppe austauscht, oder

c) — falls A eine $C_{1-6}$-Alkoxy-, $C_{1-6}$-Alkylmercapto-, Carboxyl- oder $C_{2-7}$-Alkoxycarbonyl- oder Cyanogruppe bedeutet — eine Ketocarbonsäure der Formel V

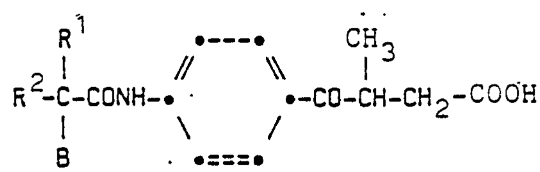

(V)

in der $R^1$, $R^2$ und B die oben angegebene Bedeutung haben, mit Hydrazin cyclisiert.

Die Umsetzung von II mit III nach dem Verfahren a) findet unter den für eine N-Acylierung üblichen Bedingungen statt, in der Regel unter Verwendung von wenigstens einer äquimolaren Menge des Säurehalogenids, zweckmäßig in Gegenwart eines Lösungsmittels, gegebenenfalls in Gegenwart eines säurebindenden Mittels, bei einer ausreichenden Temperatur von in der Regel bis zu 100 °C, bevorzugt bis zu 70 °C, gegebenenfalls bei der Siedetemperatur des Reaktionsgemisches und gegebenenfalls unter Anwendung von Druck.

Als Lösungsmittel im technischen Maßstab kommen vorzugsweise Ketone — wie Aceton, Diethylketon oder Methylethylketon-, sowie aromatische Kohlenwasserstoffe — wie Benzol, Toluol oder Xylol-, cyclische Ether — wie Tetrahydrofuran oder Dioxan-, aliphatische oder aromatische Chlorkohlenwasserstoffe — wie Methylenchlorid, Ethylenchlorid oder Chlorbenzol-, Dialkylformamide — wie Dimethylformamid — in Betracht.

Zweckmäßigerweise wird die Umsetzung in Gegenwart von säurebindenden Mitteln durchgeführt. Als solche kommen schwache anorganische Basen, wie Natrium- oder Kaliumcarbonat, Natrium- oder Kaliumhydrogencarbonat oder organische Basen, z. B. tertiäre Amine, in Frage.

Eine Herstellungmöglichkeit für die neuen Verbindungen, in denen A für einen Alkoxycarbonylrest steht, besteht darin, daß man eine Verbindung I, in der A eine Carboxylgruppe bedeutet, in üblicher Weise verestert. Die Veresterung wird zweckmäßigerweise in einem Überschuß des Alkohols als Lösungsmittel durchgeführt.

Die neuen Verbindungen (I), in denen A für eine Alkylsulfonyloxygruppe $-O-SO_2-R$ oder eine Carbaminsäureestergruppe —O—CO—NHR steht, können aus einem Alkohol, den man durch Verseifung eines Esters erhält, in üblicher Weise, d. h. durch Umsetzung mit einem Alkylsulfonsäurehalogenid $R-SO_2-Hal$ oder einem Alkansulfonsäureanhydrid $(RSO_2)_2O$, bzw. einem Isocyanat R—N=C=O, in bekannter Weise erhalten werden, wobei R für $C_1$-$C_6$-Alkyl steht.

Der Austausch des Restes X gemäß Verfahren b) gegen einen Alkoxy- oder einen Alkymercaptorest kann durch Umsetzung mit einem geeigneten Alkohol oder einem geeigneten Alkylmercaptan in Gegenwart einer Hilfsbase als säurebindendes Mittel unter an sich üblichen Bedingungen durchgeführt werden, beispielsweise unter Verwendung von wenigstens äquimolaren Mengen des Reagenzes und der Hilfsbase, zweckmäßig in Gegenwart eines Lösungsmittels, bei Temperaturen von 0 bis 80 °C, ggf. bei den Siedetemperaturen des Reaktionsgemisches, und ggf. unter Anwendung von Druck.

Als Lösungsmittel kommen unter den Reaktionsbedingungen inerte Lösungsmittel, wie aromatische Kohlenwasserstoffe, z. B. Toluol, aliphatische oder aromatische Chlorkohlenwasserstoffe, beispielsweise Methylenchlorid, Ethylenchlorid oder Chlorbenzol, Ketone, wie Aceton oder Methylethylketon, oder Dialkylformamide, wie Dimethylformamid, in Betracht. Es kann auch in dem Reagenz als Lösungsmittel gearbeitet werden.

Hilfsbasen als säurebindende Mittel sind zweckmäßigerweise anorganische Basen, wie Natriumhydrid, Natrium- bzw. Kaliumcarbonat oder Natrium- bzw. Kaliumhydrogencarbonat, oder tertiäre organische Amine, wie Triethylamin.

Der Austausch des Restes X gegen einen Alkoxyrest kann auch durch Umsetzung mit einem geeigneten Natrium- oder Kaliumalkoholat in an sich üblicher Weise durchgeführt werden, beispielsweise unter Verwendung von wenigstens einer äquimolaren Menge des Alkoholats, in einem Lösungsmittel, und zwar in dem entsprechenden Alkohol oder in einem unter den Reaktionsbedingungen inerten Lösungsmittel, wie einem aromatischen Kohlenwasserstoff, z. B. Toluol, einem offenkettigen oder cyclischen aliphatischen Ether, wie Diethylether, Tetrahydrofuran oder Dioxan, oder einem Dialkylformamid, wie Dimethylformamid, bei Temperaturen von 0 bis 100 °C, gegebenenfalls bei den Siedetemperaturen des Reaktionsgemisches, und gegebenenfalls unter Anwendung von Druck.

Der Austausch des Restes X gegen eine Nitrilgruppe kann durch Behandlung mit Natrium- oder Kaliumcyanid entweder in einem geeigneten Lösungsmittel, beispielsweise einem niederen Alkohol, wie Methanol, Ethanol oder Propanol, oder Dimethylformamid oder einem Gemisch aus Wasser und einem der oben genannten niederen Alkohole, oder in einem Zwei-Phasensystem bestehend aus Wasser und einem mit Wasser nicht mischbaren Lösungsmittel, wie einem aromatischen Kohlenwasserstoff, z. B. Toluol, oder einem aliphatischen oder aromatischen Chlorkohlenwasserstoff, z. B. Methylenchlorid, Ethylenchlorid oder Chlorbenzol geschehen. Bei der zuletzt angegebenen Arbeitsweise, der sogenannten Phasen-Transfer-Methode, ist es zweckmäßig, einen entsprechenden Katalysator, z. B. ein quartäres Ammoniumhalogenid, z. B. Tetrabutylamoniumiodid, zuzusetzen.

Die als Ausgangsstoffe verwendeten Verbindungen (IV) sind bekannt oder können unter den in den DE-OS-2 727 481 und DE-OS-2 854 475 beschriebenen Bedingungen hergestellt werden.

Die Ringschlußreaktion c) mit Hydrazin, das bevorzugt als Hydrat eingesetzt wird, geschieht vorteilhaft in einem Lösungsmittel, insbesondere einem niederen Alkohol — wie Methanol, Ethanol oder Propanol —, einem cyclischen Ether — wie Tetrahydrofuran oder Dioxan — oder in Dimethylformamid, wobei die Temperatur 50 bis 150 °C, vorzugsweise 80 bis 100 °C betragen kann. In der Regel wird hierbei pro mol (V) 1 mol Hydrazin bzw. Hydrazinhydrat verwendet.

Die Verbindungen (V) können hergestellt werden, indem man eine Aminosäure (VI),

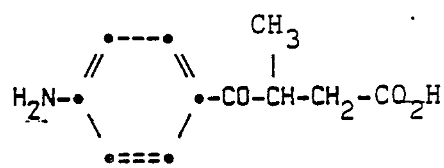

(VI)

mit III acyliert.

Die als Ausgangssubstanzen verwendeten Verbindungen der Formel II und auch der Formel VI sind bekannt (vgl. DE-OS-1 670 158, DE-OS-2 150 436 und DE-OS 2 854 475, US-PS 3 824 271 und US-PS 3 888 901) oder können unter den in den US-PS 3 324 271 und US-PS 3 888 901 beschriebenen Bedingungen hergestellt werden.

Typische Verbindungen, die nach den genannten Verfahren erhalten werden, sind beispielsweise :

6-[p-(2-Methoxy-2-methylpropionylamino) phenyl]-4,5-dihydro-5-methyl-3(H)-pyridazinon
6-[p-(2-Ethoxypropionylamino) phenyl]-4,5-dihydro-5-methyl-3(2H)-pyridazinon
6-[p-(2-Ethoxybutyrylamino) phenyl]-4,5-dihydro-5-methyl-3(2H)-pyridazinon
6-[p-(2-Propoxypropionylamino) phenyl]-4,5-dihydro-5-methyl-3(2H)-pyridazinon
6-[p-(2-Methylmercaptopropionylamino) phenyl]-4,5-dihydro-5-methyl-3(2H)-pyridazinon
6-[p-(2-Methylmercapto-2-methylpropionylamino) phenyl]-4,5-dihydro-5-methyl-3(2H)-pyridazinon
6-[p-(2-Methylmercaptobutyrylamino) phenyl]-4,5-dihydro-5-methyl-3(2H)-pyridazinon
6-[p-(2-Ethylmercaptoacetylamino) phenyl]-4,5-dihydro-5-methyl-3(2H)-pyridazinon
6-[p-(2-Ethylmercaptopropionylamino) phenyl]-4,5-dihydro-5-methyl-3(2H)-pyridazinon
6-[p-(2-Ethylmercapto-2-methylpropionylamino) phenyl]-4,5-dihydro-5-methyl-3(2H)-pyridazinon
6-[p-(2-Ethylmercaptobutyrylamino) phenyl]-4,5-dihydro-5-methyl-3(2H)-pyridazinon
6-[p-(2-Cyan-2-methylpropionylamino) phenyl]-4,5-dihydro-5-methyl-3(2H)-pyridazinon
6-[p-(2-Carboxyacetylamino) phenyl]-4,5-dihydro-5-methyl-3(2H)-pyridazinon
6-[p-(2-Carboxypropionylamino) phenyl]-4,5-dihydro-5-methyl-3(2H)-pyridazinon
6-[p-(2-Methoxycarbonylpropionylamino) phenyl]-4,5-dihydro-5-methyl-3(2H)-pyridazinon
6-[p-(2-Methylsulfonyloxyacetylamino) phenyl]-4,5-dihydro-5-methyl-3(2H)-pyridazinon
6-[p-(2-Methylaminocarbonyloxyacetylamino) phenyl]-4,5-dihydro-5-methyl-3(2H)-pyridazinon
6-[p-(2-Ethylaminocarbonyloxyacetylamino) phenyl]-4,5-dihydro-5-methyl-3(2H)-pyridazinon
6-[p-(2-Ethylaminocarbonyloxypropionylamino) phenyl]-4,5-dihydro-5-methyl-3(2H)-pyridazinon

Die Verbindungen I weisen ein asymmetrisches Kohlenstoffatom in 5-Stellung auf und werden daher normalerweise in Form ihres Racemats erhalten.

Die Erfindung betrifft auch die Enantiomeren, die vorteilhaft bereits auf der Stufe der Verbindung (II) in bekannter Weise, z. B. durch Bildung diastereomerer Salze mit einer optisch aktiven Carbonsäure, wie Dibenzoylweinsäure oder Campher-10-sulfonsäure, getrennt und durch getrennte Umsetzung der optisch aktiven Vorprodukte gewonnen werden.

Die erfindungsgemäßen Dihydropyridazinone der Formel (I) zeichnen sich durch thrombozytenaggregationshemmende und blutdrucksenkende Eigenschaften aus. Sie sind als Antihypertensiva und zur Prophylaxe und Therapie thrombotischer Erkrankungen geeignet.

Zur Untersuchung der pharmakodynamischen Eigenschaften der erfindungsgemäßen Produkte wurden folgende Methoden verwendet :

1. Hemmung der durch Collagen induzierten Aggregation von Thrombozyten der Ratte ex vivo

Die Substanzen werden Gruppen von 10 bis 15 männlichen Sprague-Dawley-Ratten (200 bis 250 g) oral appliziert. 1 Stunde nach der Applikation wird in Ether-Narkose Blut entnommen und durch Zentrifugation thrombozytenreiches Plasma gewonnen. Die photometrische Messung der Thrombozytenaggregation erfolgt unter Zusatz von $MgCl_2$ (Endkonzentration 10 mMol/l) und von Collagen Stago (Endkonzentration 0,02 mg/ml) im Born-Aggregometer Mk3. Als Aggregationsmaß findet die maximale Extinktionsänderung pro sec. Verwendung.

Als ED 33 % wird die Dosis bestimmt, welche die durch Collagen induzierte Thrombozytenaggregation um 33 % hemmt.

2. Antihypertensive Wirkung an der spontan hypertonen Ratte

Die Substanzen werden Gruppen von 4 bis 8 männlichen spontan hypertonen Okamoto-Ratten (270 bis 340 g) oral appliziert. Der systolische Blutdruck wird vor und 2 Stunden nach der Applikation unblutig mit Hilfe von Piezokristallaufnehmern gemessen.

Als ED 20 % wird unter Berücksichtigung der Werte unbehandelter Kontrolltiere die Dosis bestimmt, welche den systolischen Druck um 20 % senkt.

Die Berechnung der wirksamen Dosen erfolgte aus den linearen Beziehungen zwischen den Logarithmen der Dosen und der Wirkung mit Hilfe der Regressionsanalyse.

Als Referenzsubstanz für die Hemmung der Thrombozytenaggregation und die blutdrucksenkende Wirkung diente Amipizone (6-[p-(2-Chlorpropionylamino) phenyl]-4,5-dihydro-5-methyl-3(2H)-pyridazinon, DE-OS 2 727 481).

Die Versuche (vgl. Tabelle 1) ergaben für die erfindungsgemäßen Verbindungen bei oraler Applikation eine antihypertensive Wirkung und eine Hemmung der Thrombozytenaggregation. Die antihypertensive Wirkung tritt in 1,2- bis 6,7-mal kleineren Dosen auf als bei der Referenzsubstanz Amipizone, wobei sich das Wirkprofil verschiebt, indem sich die antihypertensive Wirkung gegenüber der thrombozytenaggregationshemmenden Wirkung verstärkt.

Tabelle 1 : Hemmung der Thrombozytenaggregation und antihypertensive Wirkung nach oraler Applikation

| Beispiel-Nr. | Hemmung der Thrombozytenaggregation 1) | | Antihypertensive Wirkung 2) | |
|---|---|---|---|---|
| | ED 33 % | R.W. | ED 20 % | R.W. |
| 1 | 1,8 | 0,35 | 1,0 | 1,20 |
| 3 | 0,85 | 0,74 | 0,36 | 3,33 |
| 8 | 5,4 | 0,12 | 1,0 | 1,20 |
| 11 | 3,0 | 0,21 | 0,18 | 6,67 |
| 13 | 0,54 | 1,12 | 0,46 | 2,61 |
| Amipizone | 0,63 | 1,00 | 1,2 | 1,0 |

1) Ratte, Appl. : per os ; ED 33 % = Dosis, welche die durch Collagen induzierte Aggregation um 33 % hemmt. R.W. = Relative Wirkung ; Amipizone = 1,00.

2) Spontan hypertone Ratte, Appl. : per os ; ED 20 % (mg/kg) = Dosis, welche den Blutdruck im Vergleich zur Kontrollgruppe um 20 % senkt. R.W. Amipizone = 1,00.

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen zu therapeutischen Zwecken bei der Behandlung von hohem Blutdruck oder thromboembolischen Erkrankungen.

Die therapeutischen Mittel oder Zubereitungen werden mit den pharmazeutisch üblichen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Dabei kommen beim Menschen Dosen von 1 bis 100, bevorzugt 5 bis 50 mg pro Patient und Tag bei oraler Gabe und von 0,1 bis 10, vorzugsweise 0,5 bis 5 mg pro Patient und Tag bei parenteraler Gabe in Betracht. Die orale Applikation ist bevorzugt.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln wie Mais, Stärke, Alginsäure oder Polyvinylpyrrolidon, Bindemitteln wie Stärke oder Gelatine, Gleitmitteln wie Magnesiumstearat oder Talkum, und/oder Mitteln zur Erzielung des Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

## Beispiel 1

6,0 g (29,5 mmol) 6-(p-Aminophenyl)-4,5-dihydro-5-methyl-3(2H)-pyridazinon und 3,9 g (35,9 mmol) Methoxyacetylchlorid wurden in 150 ml absolutem Tetrahydrofuran unter Rühren 2 h bei Rückflußtemperatur gehalten. Es wurde bei 10 °C abgesaugt, mit Wasser gewaschen und aus Dimethylformamid/Wasser umkristallisiert. Man isolierte 3,6 g (44 %) 6-[p-(Methoxyacetylamino)phenyl]-4,5-dihydro-5-methyl-3(2H)-pyridazinon als farblose Kristalle, Fp. : 212 °C.

## Beispiel 2

5,0 g (24,6 mmol) 6-(p-Aminophenyl)-4,5-dihydro-5-methyl-3(2H)-pyridazinon und 3,6 g (29,4 mmol) 2-Methoxypropionylchlorid wurden in 150 ml absolutem Tetrahydrofuran unter Rühren 2 h bei Rückflußtemperatur gehalten. Es wurde eingeengt, der Rückstand mit Wasser verrührt und abgesaugt. Nach dem Waschen mit Wasser und dem Trocknen wurde aus Propanol umkristallisiert. Man isolierte 4,0 g (56 %) 6-[p-(2-Methoxypropionylamino)phenyl]-4,5-dihydro-5-methyl-3(2H)-pyridazinon als farblose Kristalle, Fp. : 193 bis 194 °C.

## Beispiel 3

20,3 g (0,1 mol) 6-(p-Aminophenyl)-4,5-dihydro-5-methyl-3(2H)-pyridazinon wurden mit 18,1 g (0,12 mol) 2-Methoxy-butyrylchlorid und 10,1 g (0,1 mol) Triethylamin in 200 ml absolutem Aceton 3 h bei Raumtemperatur gerührt. Nach Zugabe von 100 g Eis wurde das Kristallisat abgesaugt. Nach dem Umkristallisieren aus Dimethylformamid/Wasser trocknete man bei 50 °C im Hochvakuum und erhielt 18,9 g (63 %) 6-[p-(2-Methoxybutyrylamino)phenyl]-4,5-dihydro-5-methyl-3(2H)-pyridazinon, Fp. : 151 bis 152 °C.

## Beispiel 4

20,3 g (60,1 mol) 6-(p-Aminophenyl)-4,5-dihydro-5-methyl-3(2)-pyridazinon wurden mit 14,7 g (0,12 mol) Ethoxyacetylchlorid und 400 ml absolutem Aceton 10 h bei Raumtemperatur gerührt. Es wurde bei 10 °C abgesaugt, mit kaltem Aceton gewaschen und im Vakuum bei 70 °C getrocknet. Man erhielt 14,8 g (51 %) 6-[p-(Ethoxyacetylamino)phenyl])-4,5-dihydro-5-methyl-3(2H)-pyridazinon, Fp. 195 bis 197 °C.

## Beispiel 5

20,3 g (100 mmol) 6-(p-Aminophenyl)-4,5-dihydro-5-methyl-3(2H)-pyridazinon wurden mit 18,0 g (120 mmol) 2-Acetoxy-propionylchlorid und 400 ml absolutem Aceton 10 h bei Raumtemperatur gerührt. Es wurde bei 10 °C abgesaugt, mit kaltem Aceton gewaschen und im Vakuum bei 70 °C getrocknet. Man erhielt 28,1 g (88,6 %) 6-[p-(2-Acetoxypropionyl-amino)phenyl]-4,5-dihydro-5-methyl-3(2H)-pyridazinon, Fp. : 198 bis 200 °C (Dimethylformamid/Wasser).

6,13 g (19,3 mmol) 6-[p-(2-Acetoxypropionylaminophenyl]-4,5-dihydro-5-methyl-3(2H)-pyridazinon (siehe Beispiel 1) wurden in 20 ml Methanol gelöst. Die Lösung wurde in 60 ml 1 N NaOH gegossen und 20 min bei Raumtemperatur gerührt, mit 2 N HCl auf pH 6 eingestellt, mit 100 ml Wasser versetzt und nach 2 h abgesaugt. Nach dem Absaugen und Trocknen im Vakuum bei 60 °C erhielt man 3,7 g (69,8 %) 6-[p-(2-Hydroxypropionylamino)phenyl]-4,5-dihydro-5-methyl-3(2H)-pyridazinon, Fp. : 181 bis 182 °C (n-Propanol).

13,75 g (50 mmol) 6-[p-(2-Hydroxypropionylamino)phenyl]-4,5-dihydro-5-methyl-3(2H)-pyridazinon wurden mit 3,1 g (55 mmol) Methylisocyanat und 250 ml absolutem Tetrahydrofuran unter Zusatz von 0,2 ml Triethylamin 5 h bei Raumtemperatur gerührt. Nach Absetzen des Lösungsmittels wurde der

Rückstand zwischen Essigester und Wasser verteilt. Die organische Phase wurde abgetrennt, getrocknet und eingeengt. Man erhielt nach Umkristallisieren aus Dimethylformamid/Wasser 16,6 g (69 %) 6-[p-(2-Methylaminocarbonyloxypropionylamino)phenyl]-4,5-dihydro-5-methyl-3(2H)-pyridazinon, Fp. : 96 bis 98 °C.

## Beispiel 6

27,5 g (0,1 mol) 6-p(Aminophenyl)-4,5-dihydro-5-methyl-3(2H)-pyridazinon wurden mit 13,7 g (0,12 mol) Methansulfonylchlorid und 400 ml absolutem Aceton 10 h bei Raumtemperatur gerührt. Es wurde bei 10 °C abgesaugt, mit kaltem Aceton gewaschen und im Vakuum bei 70 °C getrocknet. Man erhielt 24 g (95 %) 6-[p-(2-Methylsulfonyloxypropionyl-amino)phenyl]-4,5-dihydro-5-methyl-3(2H)-pyridazinon, Fp. 110 bis 112 °C.

## Beispiel 7

20,3 g (0,1 mol) 6-(p-Aminophenyl)-4,5-dihydro-5-methyl-3(2H)-pyridazinon wurden mit 14,9 g (0,12 mol) 2-Methylmercaptoacetylchlorid und 400 ml absolutem Aceton 10 h bei Raumtemperatur gerührt. Es wurde bei 10 °C abgesaugt, mit kaltem Aceton gewaschen und im Vakuum bei 70 °C getrocknet. Man erhielt 12,5 g (43 %) 6-[p-(2-Methylmercaptoacetylamino)phenyl]-4,5-dihydro-5-methyl-3(2H)-pyridazinon, Fp. : 220 bis 222 °C.

## Beispiel 8

Zu 6,0 g (29,5 mmol) 6-(p-Aminophenyl)-4,5-dihydro-5-methyl-3(2H)-pyridazinon in 150 ml absolutem Tetrahydrofuran wurde tropfenweise unter Rühren bei Raumtemperatur 5,5 g (53,1 mmol) Cyanacetylchlorid zugesetzt. Nach beendeter Zugabe wurde noch 10 min bei 50 °C nachgerührt. Es wurde bei 10 °C abgesaugt, zuerst mit Tetrahydrofuran, dann mit Wasser nachgewaschen und aus Dimethylformamid/Wasser umkristallisiert. Man isolierte 2,2 g (27 %) 6-[p-(Cyanacetylamino)phenyl]-4,5-dihydro-5-methyl-3(2H)-pyridazinon. 0,5 $H_2O$ als farblose Kristalle, Fp. : 263 °C (Zersetzung).

## Beispiel 9

20,3 g (0,1 mol) 6-p-(Aminophenyl)-4,5-dihydro-5-methyl-3(2H)-pyridazinon wurden mit 16,0 g (0,12 mol) 2-Cyanpropionylchlorid und 10 g (0,1 mol) Triethylamin in 200 ml absolutem Aceton 3 h bei Raumtemperatur gerührt. Nach Zugabe von 100 g Eis wurde ein Öl/Kristallgemisch isoliert. Nach dem Umkristallisieren aus Dimethylformamid/Wasser erhielt man 18,6 g (65,5 %) 6-[p-(2-Cyanpropionylamino)phenyl]-4,5-dihydro-5-methyl-3(2H)-pyridazinon. 0,2 $H_2O$ als hellgelbe Kristalle, Fp. : 130 bis 132 °C.

## Beispiel 10

20,3 g (0,1 mol) 6-(p-Aminophenyl)-4,5-dihydro-5-methyl-3(2H)-pyridazinon wurden mit 15,8 g (0,12 mol) 2-Cyanbutyrylchlorid und 10,1 g (0,1 mol) Triethylamin in 200 ml absolutem Aceton 3 h bei Raumtemperatur gerührt und nach Zugabe von 100 g Eis das Kristallisat abgesaugt. Nach dem Umkristallisieren aus Dimethylformamid/Wasser trocknete man im Hochvakuum bei 50 °C und erhielt 14,9 g (50 %) 6-[p-(2-Cyanbutyrylamino)phenyl]-4,5-dihydro-5-methyl-3(2H)-pyridazinon, Fp. : 225 bis 227 °C.

## Beispiel 11

20,3 g (0,1 mol) 6-(p-Aminophenyl)-4,5-dihydro-5-methyl-3(2H)-pyridazinon wurden mit 15,8 g (0,12 mol) 2-Cyan-2-methylpropionylchlorid in 100 ml Pyridin 3 h bei 0 °C gerührt. Nach Zugabe von 100 g Eis wurde das Kristallisat abgesaugt. Nach dem Umkristallisieren aus Dimethylformamid/Wasser und Trocknen im Hochvakuum bei 50 °C erhielt man 20,5 g (57 %) 6-[p-(2-Cyan-2-methylpropionylamino)phenyl-4,5-dihydro-5-methyl-3(2H)-pyridazinon, Fp. : 225 bis 227 °C.

## Beispiel 12

20,3 g (100 mmol) 6-(p-Aminophenyl)-4,5-dihydro-5-methyl-3(2H)-pyridazinon wurden mit 16,4 g (120 mmol) Malonsäuremonomethylesterchlorid und 400 ml absolutem Aceton 10 h bei Raumtemperatur gerührt. Es wurde bei 10 °C abgesaugt, mit kaltem Aceton gewaschen und im Vakuum bei 70 °C getrocknet. Man erhielt 15,6 g (52 %) 6-[p-(Methoxycarbonylacetylamino)phenyl]-4,5-dihydro-5-methyl-3(2H)-pyridazinon, Fp. : 228 bis 230 °C.

## Beispiel 13

2,9 g (0,01 mol) 6-[p-(2-Chlorpropionylamino)phenyl]-4,5-dihydro-5-methyl-3(2H)-pyridazinon wurden in 10 g Dimethylformamid mit 2,64 g (0,02 mol) Kaliumcyanid 4 h bei 80 °C gerührt. Nach dem Abkühlen auf Raumtemperatur wurde das Reaktionsgemisch auf 100 g Eis gegossen und das entstandene Kristallisat abgesaugt. Nach dem Umkristallisieren aus Ethanol erhielt man 1,9 g (67 %) 6-[p-(2-Cyanpropionylamino)phenyl]-4,5-dihydro-5-methyl-3(2H)-pyridazinon. 0,2 $H_2O$, Fp. : 130 bis 132 °C.

Formulierungsbeispiele

1. Tabletten

| | |
|---|---|
| Wirkstoff | 10 mg |
| Polyvinylpyrrolidon (mittl. M.G. 25 000) | 170 mg |
| Polyethylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wurde mit Polyvinylpyrrolidon in 10 %iger wäßriger Lösung befeuchtet, durch ein Sieb mit der lichten Maschenweite 1,0 mm getrieben und bei 50 °C getrocknet. Dieses Granulat wurde mit Polyethylenglykol (mittl. M.B. 4 000), Hydroxypropylmethylcellulose, Talkum und Magnesiumstearat vermischt und zu 250 mg-Tabletten verpreßt.

2. Dragees

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wurde mit einer 8 %igen wäßrigen Lösung des Polyvinylpyrrolidons durch ein 1,5 mm-Sieb granuliert, bei 50 °C getrocknet und durch ein 1,0 mm-Sieb getrieben. Das so erhaltene Granulat wurde mit Magnesiumstearat gemischt und zu Drageekernen verpreßt. Die erhaltenen Drageekerne wurden wie üblich mit einer Hülle überzogen, die im wesentlichen aus Zucker und Talkum bestand.

**Patentansprüche**

1. 6-Aryl-4,5-dihydro-3(2H)-pyridazinone der Formel

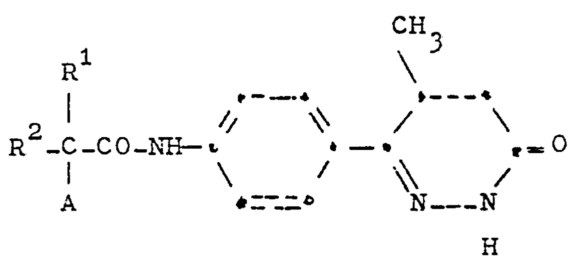

(I)

in der
$R^1$ und $R^2$ gleich oder verschieden sind und Wasserstoffatome oder $C_{1-6}$-Alkylgruppen darstellen und
A eine $C_{1-6}$-Alkoxy-, $C_{2-7}$-Alkylaminocarbonyloxy-, $C_{1-6}$-Alkylsulfonyloxy-, $C_{1-6}$-Alkylmercapto-, Carboxyl-, $C_{2-7}$-Alkoxycarbonyl oder Cyanogruppe bedeutet.

2. Verbindungen der Formel I gemäß Anspruch 1, worin
$R^1$ ein Wasserstoffatom oder eine Methylgruppe,
$R^2$ ein Wasserstoffatom oder eine Methylgruppe und
A eine Methoxy- oder Nitrilgruppe
darstellen.

3. Verfahren zur Herstellung der Verbindungen der Formel I gemäß Anspruch 1, dadurch gekennzeichnet, daß man

a) eine Verbindung der Formel II

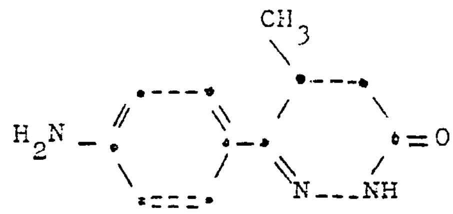

(II)

mit einem Carbonsäurehalogenid der Formel III

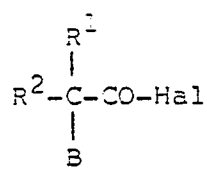

(III)

worin $R^1$ und $R^2$ die angegebene Bedeutung besitzen und B eine $C_{2-7}$-Alkanoyloxy-, $C_{1-6}$-Alkoxy-, $C_{1-6}$-Alkylmercapto-, Carboxyl-, $C_{2-7}$-Alkoxycarbonyl oder Cyanogruppe darstellt, umsetzt, in den so erhaltenen Verbindungen eine gegebenenfalls enthaltende Acyloxygruppe verseift und so erhaltene Hydroxyverbindungen mit einem reaktionsfähigen Derivat einer $C_{1-6}$-Alkylsulfonsäure oder einem $C_{2-7}$-Alkylisocyanat umsetzt oder gegebenenfalls eine Carboxylgruppe verestert, oder

b) — falls A eine $C_{1-6}$-Alkoxy-, $C_{1-6}$-Alkylmercapto- oder Cyanogruppe ist — in einer Verbindung der Formel (IV)

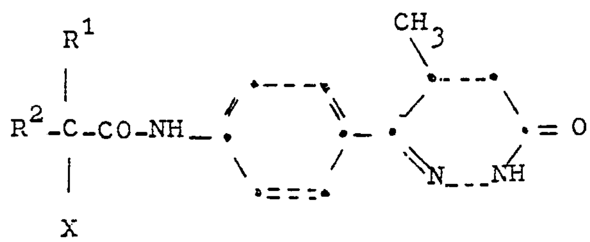

(IV)

in der $R^1$ und $R^2$ die oben angegebenen Bedeutungen haben und X für ein Chlor-, Brom- oder Iodatom oder eine Alkylsulfonyloxygruppe steht, den Rest X gegen eine $C_{1-6}$-Alkoxy-, $C_{1-6}$-Alkylmercapto- oder Cyanogruppe austauscht oder

c) — falls A eine $C_{1-6}$-Alkoxy-, $C_{1-6}$-Alkylmercapto-, Carboxyl-, $C_{2-7}$-Alkoxycarbonyl- oder Cyanogruppe bedeutet — eine Ketocarbonsäure der Formel V

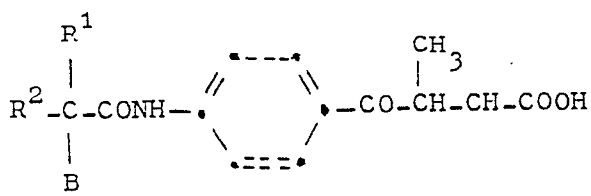

(V)

in der $R^1$, $R^2$ und B die oben angegebene Bedeutung haben, mit Hydrazin cyclisiert.

4. Arzneimittel enthaltend eine Verbindung gemäß Anspruch 1.

5. Verbindung der Formel I gemäß Anspruch 1 zur Verwendung bei der Bekämpfung von Krankheiten.

**Claims**

1. A 6-aryl-4,5-dihydro-3(2H)-pyridazinone of the formula

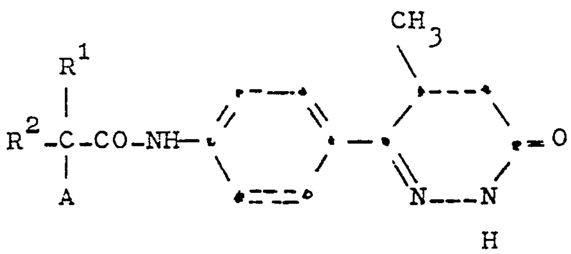

(I)

where

$R^1$ and $R^2$ are identical or different and are each hydrogen or $C_1$-$C_6$-alkyl, and

A is $C_1$-$C_6$-alkoxy, $C_1$-$C_6$-alkylmercapto, $C_2$-$C_7$-alkylaminocarbonyloxy, $C_1$-$C_6$-alkylsulfonyloxy, carboxyl, $C_2$-$C_7$-alkoxycarbonyl or cyano.

2. A compound of the formula I as claimed in claim 1, where

$R^1$ is hydrogen or methyl,

$R^2$ is hydrogen or methyl, and

A is methoxy or nitrile.

3. A process for the preparation of a compound of the formula I as claimed in claim 1, wherein

a) a compound of the formula II

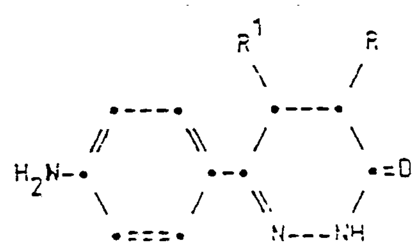

(II)

is reacted with an acyl halide of the formula III

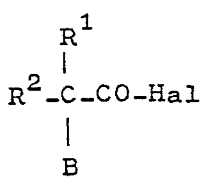

(III)

where $R^1$ and $R^2$ have the above meanings and B is $C_2$-$C_7$-acyloxy, $C_1$-$C_6$-alkoxy, $C_1$-$C_6$-alkylmercapto, carboxyl, $C_2$-$C_7$-alkoxycarbonyl or cyano, and, in the resulting compounds, a carboxyl group which may be present is hydrolyzed, and the resulting hydroxy compound is reacted with a reactive derivative of a $C_1$-$C_6$-alkylsulfonic acid or a $C_2$-$C_7$-alkyl isocyanate, or, if desired, a carboxyl group is esterified, or

b) where A is $C_1$-$C_6$-alkoxy, $C_1$-$C_6$-alkylmercapto or cyano, the radical X in a compound of the formula (IV)

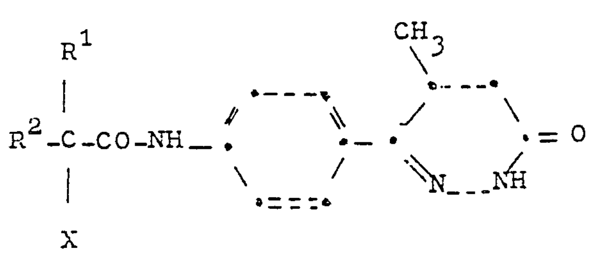

(IV)

where $R^1$ and $R^2$ have the above meanings and X is chlorine, bromine, iodine or alkylsulfonyloxy, is exchanged for a $C_1$-$C_6$-alkoxy, $C_1$-$C_6$-alkylmercapto or cyano group, or

c) where A is $C_1$-$C_6$-alkoxy, $C_1$-$C_6$-alkylmercapto, carboxyl, $C_2$-$C_7$-alkoxycarbonyl or cyano, a ketocarboxylic acid of the formula V

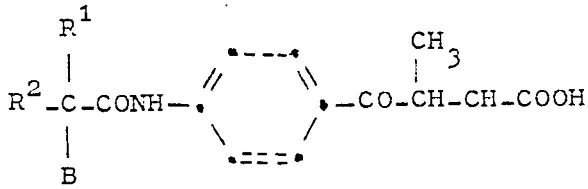

(V)

where $R^1$, $R^2$ and B have the above meanings, is cyclized with hydrazine.

4. A drug containing a compound as claimed in claim 1.
5. A compound of the formula I as claimed in claim 1 for use in the treatment of disorders.

**Revendications**

1. 6-aryl-4,5-dihydro-3(2H)-pyridazinones de formule

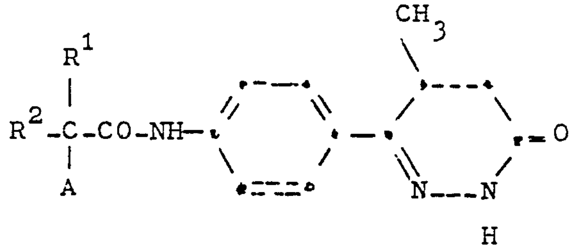

(I)

dans laquelle
$R^1$ et $R^2$ sont identiques ou différents et représentent des atomes d'hydrogène ou des groupes alkyle en $C_{1-6}$ et
A signifie un groupe alcoxy en $C_{1-6}$, alkylmercapto en $C_{1-6}$, alkylaminocarboxyloxy en $C_{2-7}$, alkylsulfonyloxy en $C_{1-6}$, carboxyle, alcoxycarbonyle en $C_{2-7}$ ou cyano
2. Composés de formule I selon la revendication 1 où
$R^1$ représente un atome d'hydrogène ou un groupe méthyle,
$R^2$, un atome d'hydrogène ou un groupe méthyle, et
A représente un groupe méthoxy ou nitrile.
3. Procédé de préparation des composés de formule I selon la revendication 1, caractérisé par le fait que :

a) on fait réagir un composé de formule II

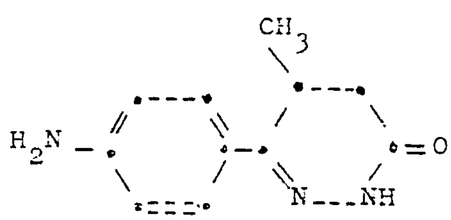

(II)

avec un halogénure d'acide carboxylique de formule III

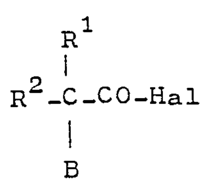

(III)

dans laquelle
$R^1$ et $R^2$ ont les significations indiquées et
B représente un groupe acyloxy en $C_{2-7}$, alcoxy en $C_{1-6}$, alkylmercapto en $C_{1-6}$, carboxyle, alcoxycarbonyle en $C_{2-7}$ ou cyano, on saponifie un groupe acyloxy éventuellement contenu dans les composés ainsi obtenus et on fait réagir les composés hydroxy ainsi obtenus avec un dérivé réactif d'un

acide alkylsulfonique en $C_{1-6}$ ou d'un alkylisocyanate en $C_{2-7}$ ou on estérifie éventuellement un groupe carboxyle, ou

b) dans le cas où A est un groupe alcoxy en $C_{1-6}$, alkylmercapto en $C_{1-6}$ ou cyano, on remplace par un groupe alcoxy en $C_{1-6}$, alkylmercapto en $C_{1-6}$ ou cyano, le reste X d'un composé de formule (IV),

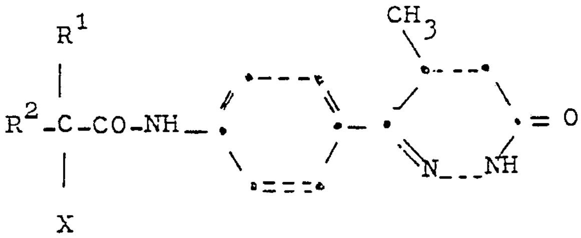

(IV)

dans lequel
$R^1$ et $R^2$ ont les significations sus-indiquées et
X représente un atome de chlore, brome ou iode ou un groupe alkylsulfonyloxy, ou

c) dans le cas où A représente un groupe alcoxy en $C_{1-6}$, alkylmercapto en $C_{1-6}$, carboxyle, alcoxycarbonyle en $C_{2-7}$ ou cyano, on cyclise avec de l'hydrazine un acide cetocarboxylique de formule V

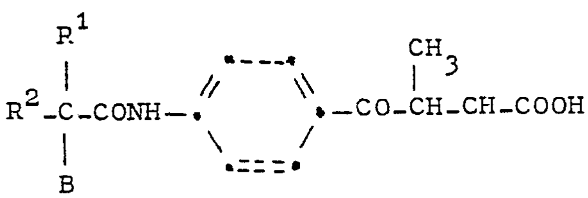

(V)

dans laquelle $R^1$, $R^2$ et B ont les significations sus-indiquées.
4. Médicament contenant un composé selon la revendication 1.
5. Composé de formule I selon la revendication 1, pour utilisation dans la lutte contre les maladies.